(19) Europäisches Patentamt
European Patent Office
Office européen des brevets

(11) Veröffentlichungsnummer: **0 302 016 A1**

(12) # EUROPÄISCHE PATENTANMELDUNG

(21) Anmeldenummer: **88810501.2**

(22) Anmeldetag: **20.07.88**

(51) Int. Cl.$^4$: **C 07 C 103/58**
C 07 C 102/00, C 08 F 20/56

(30) Priorität: **29.07.87 CH 2911/87**

(43) Veröffentlichungstag der Anmeldung:
**01.02.89 Patentblatt 89/05**

(84) Benannte Vertragsstaaten:
**BE CH DE ES FR GB IT LI**

(71) Anmelder: **CIBA-GEIGY AG**
**Klybeckstrasse 141**
**CH-4002 Basel (CH)**

(72) Erfinder: **Kuhn, Martin, Dr.**
**Dorneckstrasse 83**
**CH-4143 Dornach (CH)**

**Ouziel, Philippe**
**rue des Violettes 74**
**F-68400 Riedisheim (FR)**

(54) **N-Alkenoyl-enamide, deren Herstellung und deren Verwendung.**

㊼ Die neuen N-Alkenoyl-enamide der Formel

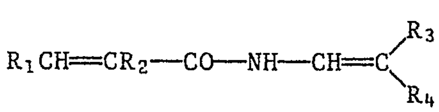

worin $R_1$ Wasserstoff oder Methyl, $R_2$ Wasserstoff, $C_1$-$C_4$-Alkyl oder Halogen und $R_3$ und $R_4$ unabhängig voneinander $C_1$-$C_4$-Alkanoyl, $C_1$-$C_{18}$-Alkoxycarbonyl, Benzoyl, Phenyl, Cyano oder Carbamoyl und $R_4$ auch Wasserstoff bedeuten, werden für in Photolacken einsetzbare, strahlenchemisch vernetzbare Massen, als UV-Absorber sowie als Textilveredelungsmittel verwendet.

EP 0 302 016 A1

**Beschreibung**

**N-Alkenoyl-enamide, deren Herstellung und deren Verwendung**

Die vorliegende Erfindung betrifft neue N-Alkenoyl-enamide, ein Verfahren zu deren Herstellung, deren Verwendung zur Herstellung neuer Polymere sowie die Verwendung der neuen Polymere in strahlenhärtbaren Massen, als UV-Absorber und als Textilveredelungsmittel.

N-Alkanoyl-enamide sind bekannt, z.B. aus DE-A 27 18 552. Sie können zur Herstellung von Acylaminen, Polymeren mit Aminogruppen oder von Aminosäure verwendet werden.

Es wurden nun neue N-Alkenoylenamide gefunden, welche zur Herstellung von Polymeren verwendet werden können, die ihrerseits in strahlenhärtbaren Massen, als UV-Absorber sowie als Textilveredelungsmittel eingesetzt werden können.

Die vorliegende Erfindung betrifft neue N-Alkenoyl-enamide der Formel

(1) 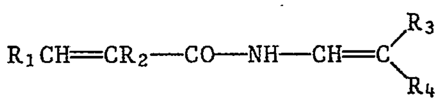

worin

$R_1$ Wasserstoff oder Methyl,
$R_2$ Wasserstoff, $C_1$-$C_4$-Alkyl oder Halogen und
$R_3$ und $R_4$ unabhängig voneinander $C_1$-$C_4$-Alkanoyl, $C_1$-$C_{18}$-Alkoxycarbonyl, Benzoyl, Phenyl, Cyano oder Carbamoyl und $R_4$ auch Wasserstoff bedeuten.

Bevorzugte Verbindungen der Formel (1) sind solche, worin $R_2$ Wasserstoff, Methyl oder Chlor und $R_3$ und $R_4$ unabhängig voneinander Acetyl, $C_1$-$C_{18}$-Alkoxycarbonyl, Benzoyl, Phenyl, Cyano oder Carbamoyl und $R_4$ auch Wasserstoff bedeuten.

Ganz besonders bevorzugt sind Verbindungen der Formel (1), worin $R_2$ Wasserstoff oder Methyl und $R_3$ und $R_4$ unabhängig voneinander Acetyl oder Ethoxycarbonyl bedeuten.

Die erfindungsgemässen N-Alkenoyl-enamide lassen sich in guten Ausbeuten und in wirtschaftlicher Weise herstellen.

Gegenstand der Erfindung ist daher auch ein Verfahren zur Herstellung dieser N-Alkenoyl-enamide der Formel (1), welches dadurch gekennzeichnet ist, dass man eine Alkenamidverbindung der Formel

(2) $R_1-CH \quad CR_2-CO-NH_2$

worin $R_1$ und $R_2$ die weiter oben angegebene Bedeutung haben, mit einem Enolether der Formel

(3) 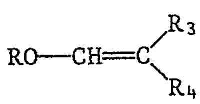

worin R $C_1$-$C_4$-Alkyl bedeutet und $R_3$ und $R_4$ die weiter oben angegebene Bedeutung haben, in Gegenwart eines Katalysators für die Alkanolabspaltung (ROH) umsetzt.

Als Katalysator für die Abspaltung des Alkanols ROH kommt besonders Phenothiazin in Betracht, das gleichzeitig als Polymerisationsinhibitor im Reaktionstemperaturbereich 130-200°C, vorzugsweise 140-190°C wirkt.

Die Alkenamidverbindungen der Formel (2) sind bekannt.

Die Enolether der Formel (3) sind zum Teil bekannt, z.B. aus US-A-2,824,121. Die neuen Enolether können nach dem in dieser US-Patentschrift beschriebenen Verfahren hergestellt werden.

Als Enolether der Formel (3) kommen z.B. Verbindungen der Formel

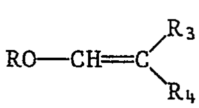

in Betracht,
worin R, $R_3$ und $R_4$ in Tabelle I angegebenen Bedeutungen haben.

Tabelle I

| Nr. | R | $R_3$ | $R_4$ | Sdp. °C/mbar | Smp. °C |
|---|---|---|---|---|---|
| a | $CH_3$ | $CH_3CO$ | $CH_3CO$ | 130-132/31,2 | |
| b | $C_2H_5$ | $CH_3CO$ | $CH_3CO$ | 136/28,6 | |
| c | $CH_3$ | $CH_3CO$ | $C_2H_5OOC$ | 142/28,6 | |
| d | $C_2H_5$ | $CH_3CO$ | $C_2H_5OOC$ | 137/20,8 | |
| e | $CH_3$ | $CH_3OOC$ | $CH_3OOC$ | 104/ 0,5 | 39,8 |
| f | $CH_3$ | CN | $C_2H_5OOC$ | 105-108/ 0,03 | 92,3-94,5 |
| g | $CH_3$ | $CH_3CO$ | H | 60-61/23,4 | |
| h | $C_2H_5$ | CN | $C_2H_5OOC$ | | 49-51 |
| i | $C_2H_5$ | CN | CN | | 65-67 |
| j | $C_2H_5$ | $C_2H_5OOC$ | $C_2H_5OOC$ | 135-140/13,0 | |
| k | $C_2H_5$ | CN | H | 188-190/ 1,3 | |

Die erfindungsgemässen N-Alkenoyl-enamide der Formel (1) können auch dadurch hergestellt werden, dass man ein Amin der Formel

(4) 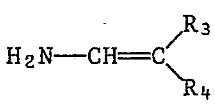

worin $R_3$ und $R_4$ die weiter oben angegebene Bedeutung haben, mit einem Säurehalogenid der Formel

(5) $R_1-CH=CR_2-CO-Hal$

worin $R_1$ und $R_2$ die weiter oben angegebene Bedeutung haben und Hal für Brom oder vorzugsweise Chlor steht, in Anwesenheit eines säurebindenden Mittels umsetzt. Als Analogverfahren ist dieses Vorgehen von L. Claisen, Liebigs Ann. Chem. 297 (1897) 16 bis 32, beschrieben

Als Amine der Formel (4) sind z.B. Verbindungen zu erwähnen, worin $R_3$ und $R_4$ folgende Bedeutungen einnehmen:

Tabelle II

| Nr. | $R_3$ | $R_4$ | Smp. °C | Sdp. °C/mbar |
|---|---|---|---|---|
| a | CN | CN | 127 | |
| b | CN | $COOC_2H_5$ | 130 | |
| c | $COCH_3$ | $COCH_3$ | 144 | |
| d | $COCH_3$ | $COOC_2H_5$ | 47 | |
| e | $COOCH_3$ | $COOCH_3$ | 125 | |
| f | $COCH_3$ | H | | 46/0,06 |
| g | CN | H | | |

Als Säurehalogenide der Formel (5) kommen z.B. das Chlorid der Acryl-, Methacryl- oder Crotonsäure in Betracht.

Die Reaktion verläuft exotherm, häufig bereits bei Zimmertemperatur, unter Bildung von Salzsäure.

Die erfindungsgemässen N-Alkenoyl-enamide der Formel (1) können zur Herstellung von Polymeren verwendet werden, die 100-1 Mol % an Struktureinheiten der Formel I und 0-99 Mol % an Struktureinheiten

der Formel II enthalten

(I) 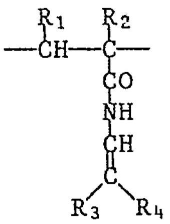 , (II) 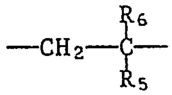

worin

$R_1$, $R_2$, $R_3$ und $R_4$ die oben angegebenen Bedeutungen haben, $R_5$ für Carboxy, $C_1$-$C_{18}$-Alkoxycarbonyl, N,N-Di-$C_1$-$C_4$-alkylamino-$C_1$-$C_4$-alkylcarbamoyl Cyano, Formyl, N,N-Di-$C_1$-$C_4$-alkylamino-$C_1$-$C_4$-alkoxycarbonyl, $C_1$-$C_4$-Alkanoyloxy, unsubstituiertes oder substituiertes Carbamoyl, 1-Pyrrolidonyl, 1-Imidazolyl, 2- bzw. 4-Pyridinyl oder Phenyl und $R_6$ für Wasserstoff oder Methyl stehen, indem man sie in Gegenwart eines organischen inerten Lösungsmittels und eines Polymerisationsinitiators bei erhöhter Temperatur einer radikalinduzierten Polymerisation unterwirft.

Als organische inerte Lösungsmittel sind protische und aprotische Lösungsmittel, wie z.B. Tetrahydrofuran, Dioxan, Toluol, Xylol, Ethylacetat, Butylacetat, 4-Butyrolacton, Aceton, Diethylketon, Methylethylketon, Methylisobutylketon, Methylglykolacetat, Ethylglykolacetat, Dimethylformamid, Dimethylacetamid und N-Methylpyrrolidon zu erwähnen.

Als Polymerisationsinitiatoren kommen Verbindungen mit thermisch leicht homolytisch spaltbaren Bindungen in Betracht, z.B. Hydroperoxide, Peroxide, Perester, wie sie von M. Warson, Fa. Chandler, Braintree/England "Per-Compounds and Per-Salts in Polymer Processes", (1980), 161 ff. (C.A. 93, (1980) 205312 v) beschrieben sind, sowie aliphatische Azoverbindungen wie Azobisisobutyronitril, 4,4'-Azo-bis-4-cyanovaleriansäureester u.a..

Die Polymerisation erfolgt bei Temperaturen zwischen 60 und 100°C.

Als Copolymer-Bestandteil der Formel II kommen Vinylverbindungen, wie Acryl- und Methacrylsäure, Alkylester und Alkylaminoalkylamide der Acryl-und Methacrylsäure, Styrol, N-Di-$C_1$-$C_4$-alkylamino-$C_1$-$C_4$-alkylester der (Meth)Acrylsäure, (Meth)Acrylamid, N-substituiertes (Meth)Acrylamid, N-Di-$C_1$-$C_4$-alkylamino-$C_1$-$C_4$-alkyl-(meth)acrylamide, (Meth)Acrylnitril, Acrolein, Acroleinderivate, Vinylacetat, 1-Vinylpyrrolidon, 1-Vinylimidazol und 2- bzw. 4-Vinylpyridin.

Die Polymere, die erfindungsgemäss erhalten werden können, stellen daher einen weiteren Gegenstand der vorliegenden Erfindung. Sie entsprechen der weiter oben angegebenen Formel I + II.

Diese Polymere, d.h. die Copolymere und Homopolymere, können in photovernetzbaren Massen zur Erzeugung von Abbildungen wie Fotolacken, zur Ausbildung von Reliefmustern im gewerblichen Druck, zur Erzeugung von Resists hoher Auflösung zur Anwendung im mikroelektronischen Bereich, in UV-härtbaren Ueberzügen und Beschichtungsmassen hoher Lösemittel- und Temperaturbeständigkeit, im Hinblick darauf, dass diese Polymere im Bereich 270-300 nm absorbieren als UV-Absorber in UV-absorbierenden Folien wie Sandwich-Gläsern für Kraftfahrzeuge und Gebäudeverglasung und in Schutzfolien für Treibhäuser oder als Textilveredelungsmittel zur Verleihung bestimmter Eigenschaften wie Knitterfestigkeit und Wasserfestigkeit verwendet werden.

Die Copolymere gestatten besonders die Herstellung thermisch beständiger Resistbilder mit guter Auflösung, da sie im allgemeinen relativ hohe Glasübergangstemperaturen ($T_g$), kombiniert mit guten mechanischen Eigenschaften, aufweisen.

Die folgenden Beispiele erläutern die Erfindung näher.

## Herstellung der Monomere

### Beispiel 1:

In einem Rundkolben werden 252 g Ethoxymethylen-acetylaceton, 114,7 g Acrylamid und 1 g Phenothiazin vorgelegt und das Ganze unter Rühren auf 160°C erhitzt. Bei 140°C beginnt das sich abspaltende Ethanol abzudestillieren. Nach Abtreiben von 64,3 g Ethanol unterbricht man (durch Kühlung) die Reaktion und destilliert danach das gebildete Monomer ab.

Das erhaltene Produkt (251 g) wird aus 220 ml Isopropanol umkristallisiert und man erhält 188 g (64 % d. Th.) des Monomers der Formel

(100) 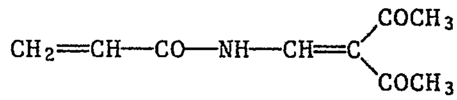

mit einem Smp. von 58,6-59,8°C.

Beispiel 2:

In einem Rundkolben werden 377,7 g Ethoxymethylen-acetoethylacetat, 144,2 g Acrylamid und 5 g Phenothiazin vorgelegt und das Ganze auf 160°C erhitzt. Nach Abtreibung von 84 g Ethanol unterbricht man (durch Kühlung) die Reaktion und destilliert anschliessend das gebildete Monomer ab. Nach Umkristallisation aus 300 ml Isopropanol erhält man 275 g des Monomers der Formel

(200) 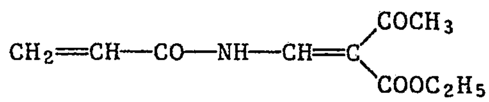

mit einem Smp. von 65,2-66,8°C.

Beispiel 3:

Analog dem Verfahren von Beispiel 1 lässt man bei 190°C 350,5 g Ethoxymethylen-malonsäure-diethylester, 138,5 g Acrylamid und 4 g Phenothiazin reagieren. Nach Destillation von 60 g Ethanol, Abdestillieren des gebildeten Monomers und Umkristallisation aus 110 ml Cyclohexan erhält man 90 g (23 % d.Th.) des Monomers der Formel

(300) 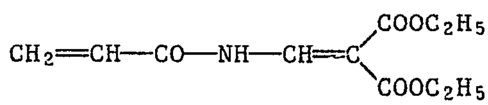

das nach erneuter Destillation einen Smp. von 46,2-47,2°C aufweist.

Beispiel 4:

Analog dem Verfahren von Beispiel 1 lässt man bei 155°C 217,6 g Ethoxymethylen-aceto-ethylacetat, 99,5 g Methacrylamid und 2,5 g Phenothiazin reagieren. Man erhält 171 g (65 % d.Th.) des Monomers der Formel

(400) 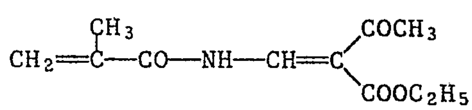

vom Smp. 25,5°C.

In analoger Weise erhält man folgende Monomere:

(401) 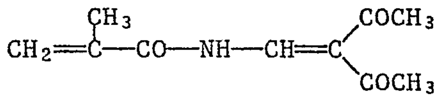

Smp. 46,8-47,9°C

(402) 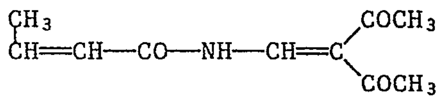

Smp. 33,2-34,7°C

(403) 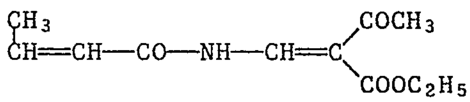

Smp. 90,4-91,9°C.

(404) 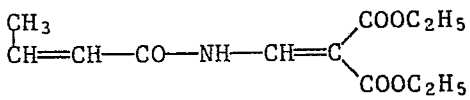

Smp. 68,5-69,8°C

(405) 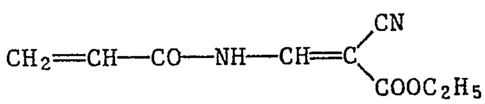

Smp. 66,3-68,5°C.

Herstellung der Polymere

Beispiel 5:

In einem Reaktionsgefäss, versehen mit Rührer, Kühler und Thermometer, werden 18,75 g des Monomers der Formel (200), 0,02 g tert.-Butyl-peroxi-2-ethyl-hexanoat und 28 g Dioxan vorgelegt. Nach 10-minütigem Einleiten von Stickstoff wird die Mischung auf 82°C aufgeheizt. Ab 77°C setzt die Polymerisation unter Wärmetönung ein und nach 90 Minuten tritt eine leichte Trübung ein. Nach 2 Stunden wird die Reaktion durch Abkühlen unterbrochen und die leicht trübe, viskose Lösung mit 35 ml Dioxan verdünnt. Das Homopolymer wird durch Eingiessen der Lösung in Ethanol ausgefällt. Man erhält 17,1 g (91 % d.Th.) des Homopolymers mit einer reduzierten Viskosität von 0,32 dl/g in Dioxan (c = 0,5 % g/V).

Beispiel 6:

Man verfährt wie in Beispiel 5 beschrieben, verwendet jedoch 39 g des Monomers der Formel (100), 0,2 Didodecanoylperoxid und 47 ml Tetrahydrofuran. Nach dem Verdrängen der Luft mit Stickstoff wird die Mischung während 20 Stunden unter Rühren auf 67°C erhitzt. Die Lösung wird schwach trüb und viskos. Man lässt abkühlen und fällt dann das Homopolymer durch Eingiessen der Lösung in Ethylalkohol. Nach dem Trocknen erhält man 38,2 g (97,4 % d.Th.) des Homopolymers mit einer reduzierten Viskosität von 0,10 dl/g in Aceton (c = 0,5 % g/V).

Beispiel 7:

Man verfährt wie in Beispiel 5 beschrieben, unter Verwendung jedoch von 25,4 g des Monomers der Formel (100), 6 g Ethylacrylat und 0,1 g tert.-Butyl-peroxi-2-ethyl-hexanoat und 90 ml Essigester. Nach Verdrängung der Luft mit Stickstoff wird die Mischung während 5 Stunden unter Rühren auf 76°C erhitzt. Man lässt abkühlen und fällt danach das Copolymer durch Eingiessen der Lösung in Ethylalkohol. Nach dem Trocknen erhält man 28,8 g des Polymers, bestehend aus 70 Mol % des Monomers der Formel (100) und 30 Mol % Ethylacrylat, mit einer reduzierten Viskosität von 0,41 dl/g in Aceton (c = 0,5 % g/V).

Beispiel 8:

Man verfährt wie in Beispiel 5 beschrieben, verwendet aber 16,5 g des Monomers der Formel (401), 1,1 g Acrylsäure, 0,04 g tert.-Butyl-peroxi-2-ethyl-hexanoat und 26 g Dioxan. Nach dem Verdrängen der Luft mit Stickstoff wird die Mischung während 3 1/4 Stunden unter Rühren auf 82°C erhitzt. Man lässt abkühlen und giesst danach die Lösung in Ethanol ein, worauf das Polymer ausfällt. Nach dem Trocknen erhält man 14 g (79,4 % d.Th.) des Polymers, bestehend aus 84,6 Mol % des Monomers der Formel (401) und 15,4 Mol % Acrylsäure, mit einer reduzierten Viskosität von 0,28 dl/g in Aceton (c = 0,5 % g/V).

In analoger Weise erhält man aus dem Monomer der Formel

(500) 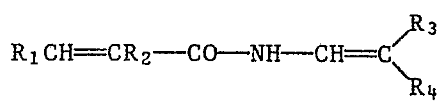

worin $R_1$ Wasserstoff und $R_3$ und $R_4$ $-COCH_3$ sind und $R_2$ die in Tabelle I angegebene Bedeutung hat und gegebenenfalls dem Comonomer Acrylsäure (A), Ethylacrylat (B) oder Styrol (C) folgende Polymere

Tabelle I

| Nr. | $R_2$ | Comonomer | Monomer (Mol %) | Comonomer (Mol %) | Initiator (Mol %) | Ausbeute (%) | $\eta^*_{red}$ (g/dl) |
|---|---|---|---|---|---|---|---|
| 1 | H | - | 99,90 | - | 0,1 | 94 | 0,79 |
| 2 | H | - | 99,90 | - | 0,1 | 78,3 | 0,23 |
| 3 | H | B | 49,90 | 49,82 | 0,27 | 92 | 0,99 |
| 4 | H | B | 89,75 | 9,94 | 0,3 | 96,5 | 0,36 |
| 5 | H | A | 84,68 | 15,20 | 0,11 | 88,5 | 0,22 |
| 6 | H | C | 49,77 | 49,81 | 0,41 | 90 | 0,30 |
| 7 | $CH_3$ | - | 99,80 | - | 0,20 | 72 | 0,30 |

* gemessen in Aceton bei c=0,5 % g/v

Lösungsmittel zur Polymerisation:
Ethylacetat für Nr. 1, 3 und 4
Dioxan für Nr. 2, 5 und 7
Toluol für Nr 6

Beispiel 9:

In einem mit Rührer, Kühler unt Thermometer versehenen Reaktionsgefäss werden 30 g des Monomers der Formel (200), 14,2 g Ethylacrylat, 0,15 g tert.-Butyl-peroxi-2-ethyl-hexanoat und 100 ml Ethylacetat zugegeben. Die Mischung wird unter Rühren während 4 Stunden auf 75° C erhitzt. Nach Abziehen des Lösungsmittels und Wiederaufnahme des gebildeten Copolymers in Aceton, wird das Polymer durch Fällen in Wasser isoliert. Man erhält 41,1 g (92,7 % d.Th.) Copolymer bestehend aus 50 Mol % Einheiten des Monomers der Formel (200) und 50 Mol % Ethylacrylat.

Beispiel 10:

Man verfährt analog zu Beispiel 9, verwendet jedoch 23,8 g des Monomers der Formel (200), 3,4 g Dimethylaminopropyl-methacrylamid, 0,06 g tert.-Butyl-peroxi-2-ethyl-hexanoat und 40 g Dioxan. Die Mischung wird unter Rühren während 5 Stunden auf 83° C erhitzt, mit weiteren 0,06 g tert.-Butyl-peroxi-2-ethyl-hexanoat versetzt und während 14 Stunden bei 83° C polymerisiert. Man lässt dann abkühlen und fällt die Lösung in Ethylalkohol worauf das Copolymerisat ausfällt. Man erhält 11,2 g (41 % d.Th.) eines Copolymerisats bestehend aus 85 Mol % Einheiten der Formel (200) und 15 Mol % Dimethylaminopropyl-methacrylamid.

In analoger Weise erhält man aus dem Monomer der Formel (500), worin $R_1$ Wasserstoff, $R_3$ $-COCH_3$ und $R_4$ $-COOC_2H_5$ sind und $R_2$ die in Tabelle II angegebene Bedeutung hat, und gegebenenfalls dem Comonomer (A) oder (C) folgende Polymere

Tabelle II

| Nr. | $R_2$ | Comonomer | Monomer (Mol %) | Comonomer (Mol %) | Initiator (Mol %) | Ausbeute (%) | $\eta^*_{red}$ (g/dl) |
|---|---|---|---|---|---|---|---|
| 8 | H | - | 99,76 | - | 0,24 | 95,6 | 0,107 |
| 9 | H | - | 99,81 | - | 0,19 | 95,8 | 0,416 |
| 10 | H | - | 99,90 | - | 0,10 | 91 | 0,29 |
| 11 | H | C | 49,68 | 49,84 | 0,48 | 93,6 | - |
| 12 | H | A | 84,62 | 15,26 | 0,11 | 88 | 0,34 |
| 13 | $CH_3$ | - | 99,11 | - | 0,89 | 85,6 | 0,14 |
| 14 | $CH_3$ | - | 99,80 | - | 0,20 | 72 | 0,44 |
| 15 | $CH_3$ | A | 84,53 | 15,29 | 0,18 | 78,6 | 0,32 |

* gemessen in Aceton bei c=0,5 % g/V

Lösungsmittel zur Polymerisation:
Tetrahydrofuran für Nr. 8
Ethylacetat für Nr 9 und 13
Dioxan für Nr. 10, 12, 14 und 15
Toluol für Nr. 11

Beispiel 11:

Man verfährt analog zu Beispiel 9, verwendet jedoch 20,4 g des Monomers der Formel (300), 1,1 g Acrylsäure, 0,04 g tert.-Butylperoxi-2-ethyl-hexanoat und 32 g Dioxan. Die Mischung wird unter Rühren während 2 1/2 Stunden auf 82°C erhitzt. Nach dem Abkühlen giesst man die Lösung in Wasser, worauf das Copolymerisat ausfällt. Man erhält 20,5 g (95,3 % d.Th.) eines Mischpolymerisats, bestehend aus 84,6 Mol % Monomer der Formel (300) und 15,4 Mol % Acrylsäure mit einer reduzierten Viskosität von 0,3 dl/g in Aceton (c = 0,5 % g/V).

In analoger Weise erhält man:

- aus dem Monomer der Formel (500), worin $R_1$ und $R_2$ Wasserstoff und $R_3$ und $R_4$ $-COOC_2H_5$ sind, folgende Homopolymere

Tabelle III

| Nr. | Comonomer | Monomer (Mol %) | Comonomer (Mol %) | Initiator (Mol %) | Ausbeute (%) | $\eta^*_{red}$ (g/dl) |
|---|---|---|---|---|---|---|
| 16 | - | 99,74 | - | 0,26 | 100 | 0,100 |
| 17 | - | 99,66 | - | 0,34 | 92,2 | 0,702 |
| 18 | - | 99,80 | - | 0,20 | 96,7 | 0,313 |

* gemessen in Aceton bei c=0,5 % g/V

Lösungsmittel zur Polymerisation:

Tetrahydrofuran Für Nr. 16

Ethylacetat für Nr. 17

Dioxan für Nr. 18

- aus dem Monomer der Formel (500), worin $R_1$ Methyl, $R_2$ Wasserstoff und $R_3$ und $R_4$ die in Tabelle IV angegebenen Bedeutungen haben und dem Comonomer Vinylacetat (E) folgende Polymere

Tabelle IV

| Nr. | $R_3$ | $R_4$ | Comonomer | Monomer (Mol %) | Comonomer (Mol %) | Initiator (Mol %) | Ausbeute (%) | $\eta^*_{red}$ (g/dl) |
|---|---|---|---|---|---|---|---|---|
| 19 | $CH_3-CO-$ | $C_2H_5-COO-$ | E | 49,88 | 49,60 | 0,67 | 90 | 0,27 |
| 20 | $C_2H_5-COO-$ | $C_2H_5-COO-$ | E | 49,81 | 49,67 | 0,68 | 61 | 0,08 |
| 21 | $CH_3-CO-$ | $CH_3-CO-$ | E | 49,83 | 49,64 | 1,2 | 31 | 0,07 |

* gemessen in Aceton bei c=0,5 % g/V

Lösungsmittel zur Polymerisation: Dioxan.

Beispiel 12:

In einen mit Rührer, Thermometer und Rückflusskühler (Ausgang gesperrt durch Blasenzähleraufsatz mit Dünnparaffinfüllung) versehenen 50 ml Planschliffkolben werden 15,0 g Acrylamidomethylenacetylaceton, 3,8 g N-tert.butyl-acrylamid, 2,7 g Acrylsäure, 1,0 g Tert.butyl-peroctoat (10%ig in Dioxan) und 31,0 g Dioxan. Der Monomergehalt der Lösung beträgt 40,2 Gew.%. Nach sorgfältigem Spülen mit $N_2$ wird der Ansatz unter Rühren auf 80°C Innentemperatur aufgeheizt. In stark exothermer Reaktion, bei der die Innentemperatur bis auf 105°C ansteigt, läuft die Polymerisation ab. Nach 30 Minuten wird mit 5 ml Dioxan verdünnt und 5 Stunden bei 80°C auspolymerisiert. Man erhält eine hochviskose, klare Lösung, die nach weiterem Verdünnen mit Dioxan in Wasser eingefällt wird. Nach dem Trocknen erhält man 20,6 g (95,8 % d.Th.) eines farblosen Mischpolymerisats mit einem Erweichungspunkt >200°C und einer reduzierten Viskosität von 0,73 dl/g in Dioxan.

Beispiel 13:

Man verfährt analog zu Beispiel 12, verwendet jedoch 15,0 g Acrylamidomethylen-acetylaceton, 3,5 g N-Methyl-N-vinyl-acetamid, 0,7 g Tert.butyl-peroctoat (10%ig in Dioxan) und 27,0 g Dioxan. Der Monomergehalt der Lösung beträgt 40 Gew.%. Nach sorgfältigem Spülen mit $N_2$ wird der Ansatz unter Rühren auf 80°C Innentemperatur aufgeheizt. Unter Ansteigen der Temperatur bis 98°C läuft in stark exothermer Reaktion die Polymerisation ab. Man verdünnt mit 20 ml Dioxan und polymerisiert 5 Stunden bei 82°C aus. Die entstehende klare, stark viskose Lösung von leicht gelber Farbe wird nach weiterem Verdünnen mit Dioxan in Wasser eingefällt. Nach dem Trocknen erhält man 17,8 g (96,2 % d.Th.) eines praktisch farblosen Mischpolymerisats mit einem Erweichnungspunkt >200°C. Das Material verliert nach dem Trocknen seine Löslichkeit in Dioxan sowie DMA und ist nur noch stark quellbar.

Beispiel 14:

Man verfährt analog zu Beispiel 12, verwendet jedoch 10,0 g Acrylamidomethylen-acetylaceton, 2,9 g Styrol, 2,8 g Aethylacrylat, 0,7 g Tert. butyl-peroctoat (10%ig in Dioxan) und 23,0 g Dioxan. Der Monomergehalt beträgt 39,9 Gew.%. Nach sorgfältigem Spülen mit $N_2$ wird der Ansatz unter Rühren auf 80°C aufgeheizt. Man hält 5 Stunden bei dieser Temperatur und fällt den Ansatz nach Verdünnen mit Dioxan sodann in Wasser ein. Man erhält 15,0 g (95,5 % d.Th.) eines farblosen Mischpolymerisats mit einem Erweichungspunkt von 158°C und einer reduzierten Viskosität von 0,72 dl/g in Dioxan.

Beispiel 15:

Man verfährt analog zu Beispiel 12, verwendet jedoch 11,1 g Acrylamidomethylen-acetessigsäureäthylester, 0,85 g Acrylnitril, 1,3 g N-Vinylpyrrolidon-(2), 0,4 g Tert.butyl-peroctoat (10%ig in Dioxan) sowie 24,0 g Dioxan. Man spült sorgfältig mit $N_2$ und heizt den Ansatz unter Rühren auf 80°C auf. Nach Abklingen der exotherm verlaufenen Polymerisationsreaktion hält man noch 5 Stunden auf 80°C und fällt sodann die klare, stark viskose Lösung nach weiterem Verdünnen mit Dioxan in Wasser ein. Nach dem Trocknen erhält man 12,2 g (92 % d.Th.) eines farblosen Copolymerisats mit einem Erweichnungspunkt von 170°C und einer reduzierten Viskosität von 0,93 dl/g in Dioxan.

Beispiel 16:

Man verfährt analog zu Beispiel 12, verwendet jedoch 13,0 g Acrylamidomethylen-acetessigsäureäthylester, 2,0 g N-Methoxymethyl-methacrylamid, 0,4 g Tert.butyl-peroctoat (10%ig in Dioxan) sowie 27,0 g Dioxan. Der Monomergehalt der Lösung beträgt 35,4 Gew.% Man spült mit $N_2$ und heizt auf 80°C Innentemperatur auf. Nach Abklingen der exotherm verlaufenden Polymerisation hält man noch 5 Stunden bei 80°C und fällt sodann den Ansatz nach Verdünnen mit Dioxan in Wasser ein. Man erhält, nach dem Trocknen, 14,4 g (96 % d.Th.) eines farblosen Copolymersats, das einen Erweichungspunkt von 158°C und eine reduzierte Viskosität von 0,3 dl/g in Dioxan hat.

Beispiel 17:

Man verfährt analog zu Beispiel 12, verwendet jedoch 13,0 g Acrylamidomethylen-acetessigsäureäthylester, 1,3 g Vinylacetat, 0,4 g Tert. butyl-peroctoat (10%ig in Dioxan) sowie 47 g Dioxan. Der Monomergehalt der Lösung liegt bei 23,2 Gew.%. Man spült sorgfältig mit $N_2$ und polymerisiert 2 Stunden bei 80°C. Die erhaltene klare, viskose Lösung wird nach Verdünnen mit Dioxan in Wasser eingefällt. Nach dem Trocknen erhält man 13.2 g (92,3 % d.Th.) eines farblosen Copolymerisats mit einem Erweichungspunkt von 165°C und einer reduzierten Viskosität von 0,26 dl/g in Dioxan.

Beispiel 18:

Man verfährt analog zu Beispiel 12, verwendet jedoch 20,0 g Acrylamidomethylen-acetylaceton, 10,7 g Stearylmethacrylat, 2,0 g N-tert.butylacrylamid, 0,1 g Tert.butyl-peroctoat und 60 g Dioxan. Der Monomergehalt beträgt 35 Gew.%. Nach sorgfältigem Spülen mit $N_2$ wird der Ansatz unter Rühren auf 80°C Innentemperatur aufgeheizt. Man hält 4,5 Stunden auf dieser Temperatur, verdünnt sodann die viskose, klare, leicht gelbe Lösung mit 50 ml Dioxan und fällt in Wasser ein. Man erhält 31,9 g (97,2 % d.Th.) eines farblosen Mischpolymerisats mit einem Erweichungspunkt von 97°C und einer reduzierten Viskosität von 0,38 dl/g in Dioxan.

Beispiel 19:

12,0 g Acrylamidomethylen-acetylaceton, 0,9 g Acrylnitril, 28,0 g Stearylmethacrylat, 0,1 g Tert.butyl-peroctoat und 65 ml Dioxan werden miteinander eingewogen. Der Monomergehalt beträgt 39 Gew.%. Nach sorgfältigem Spülen mit $N_2$ wird der Ansatz unter Rühren auf 80°C Innentemperatur aufgeheizt und 5 Stunden bei dieser Temperatur auspolymerisiert. Die viskose, klare, gelbe Lösung wird mit weiterem Dioxan verdünnt und der Ansatz sodann in 2 Liter Wasser eingefällt. Man erhält 40 g (97,5 % d.Th.) eines farblosen Mischpolymerisats mit einem Erweichungspunkt von 55°C und einer reduzierten Viskosität von 0,42 dl/g in Dioxan.

Beispiel 20:

12,0 g Acrylamidomethylen-acetylaceton, 1,8 g Acrylnitril, 22,4 g Stearylmethacrylat, 0,1 g Tert.butyl-peroctoat und 60 ml Dioxan werden miteinander eingewogen. Der Monomergehalt beträgt 37 Gew.%. Nach sorgfältigem Spülen mit $N_2$ wird der Ansatz unter Rühren auf 80°C Innentemperatur aufgeheizt und 5 Stunden bei dieser Temperatur auspolymerisiert. Die viskose, klare, gelbe Lösung wird anschliessend mit Dioxan verdünnt und der Ansatz sodann in 2 Liter Wasser eingefällt. Man erhält 34,8 g (96 % d.Th.) eines farblosen Mischpolymerisats mit einem Erweichungspunkt von 60°C und einer reduzierten Viskosität von 0,55 dl/g in Dioxan.

Anwendungsbeispiele

Beispiel 21: Beschichtungslösung
Polymer Nr. 3 aus Tabelle I   1,5 g
4-Butyrolacton   10,0 g
Netzmittel auf Basis von Perfluorcarbonsäure z.B. Fluorad® FC 430 der 3M   0,001 g

Die Lösung wird mittels eines Ziehrakels in 12 μ Filmdicke auf eine kupferkaschierte Epoxyharzplatte beschichtet. Man trocknet während 2 1/2 Stunden bei 60°C, überzieht die Platte mit einer Photomaske (Stouffer-Keil) und belichtet diese Platte aus 18 cm Entfernung während 300 Sekunden mit einer 1000 W UV-Lampe. Die Platte wird dann entwickelt. Die letzte klar abgebildete Stufe war 9.

Beispiel 22:

Baumwoll-Cretonne wird mit Flotten folgender Zusammensetzung
(A) 100 g des Polymerisates des Beispiels 12, 13, 15, 16 oder 17,
900 g Dioxan und
0,01 g eines Sensibilisators.
(B) 100 g des Polymerisates des Beispiels 12, 13, 15, 16 oder 17
900 g Dioxan,
0,01 g eines Sensibilisators und
0,05 g eines Photoinitiators imprägniert, auf eine Flottenaufnahme von 100 % abgequetscht und danach 4 Stunden bei 60°C getrocknet.

Die so behandelten Cretonne-Muster werden nun auf ihre Eignung im Textilfinishing geprüft, indem man die Muster

a) zwei Mal in Dioxan extrahiert,

b) mit einer Staub-UV-Lampe von 5000 W (W. Staub GmbH, Neu-Isenburg, Deutschland) in einem Abstand von 80 cm während 5 Minuten einseitig bestrahlt und

c) wie unter b) bestrahlt und dann zwei Mal mit Dioxan extrahiert, und anschliessend den $N_2$-Gehalt in % bestimmt.

Die Resultate sind in der nachfolgenden Tabelle V zusammengestellt:

Tabelle V

| Polymer von Beispiel | Flotte | getrocknet extrahiert % $N_2$ * | getrocknet UV-bestrahlt % $N_2$ * | getrocknet UV-bestrahlt extrahiert % $N_2$ * |
|---|---|---|---|---|
| 12** | A | 0,01 | 1,06 | 0,42 |
| | B | 0,02 | 0,96 | 0,43 |
| 13 | A | 0,51 | 1,06 | 1,08 |
| | B | 0,41 | 1,12 | 0,89 |
| 15 | A | 0,71 | 1,26 | 0,79 |
| | B | 0,73 | 1,21 | 1,02 |
| 16 | A | 0,04 | 1,16 | 0,92 |
| | B | 0,02 | 1,16 | 0,96 |
| 17 | A | 0,05 | 0,94 | 0,75 |
| | B | 0,05 | 0,97 | 0,88 |

* Elementaranalyse

** Dieses Muster wurde nicht extrahiert, sondern im Flottenverhältnis 1:40, während 30 Minuten bei 95°C in einer Flotte enthaltend pro Liter 5 g Seife und 2 g Natriumcarbonat gewaschen und dann getrocknet.

Beispiel 23:

Eine 33/67 Baumwolle/Diolen-Mischgewebe (210 g/m²) wird mit einer Flotte enthaltend
50 g des Polymerisates von Beispiel 18,
950 g Dioxan und
0,01 g eines Sensibilisators
imprägniert, auf eine Flottenaufnahme von 80 % abgequetscht und danach bei 60°C getrocknet. Das behandelte trockene Gewebe wird nun beidseitig wie in Beispiel 22 beschrieben, bestrahlt und anschliessend zwei Mal mit Dioxan extrahiert, um gegebenenfalls nicht reagiertes Polymerisat zu entfernen. Eine Prüfling von 20 x 25 cm wird bei 20° C und 65 % relativer Luftfeuchtigkeit konditioniert und gewogen (Gewicht A) und dann dem Spray-Test unterzogen, indem man ihr in 2 Klammern entspannt und mit 500 ml Wasser von 20°C beregnet und drei Mal kräftig in der Längsrichtung anspannt, um ihn von anhaftenden Tropfen zu befreien. Die Gewebeprobe wird dann je an einer Seite gefasst, dreimal kräftig ausgeschlagen und gewogen (Gewicht B). Aus den Gewichten wird nun die aufgenommene Wassermenge bestimmt

$$(\%\ \text{Wassermenge} = \frac{B-A}{A} \cdot 100).$$

Der gleiche Test wird an einer nicht ausgerüsteten und an einer ausgerüsteten aber nicht bestrahlten Gewebeprobe durchgeführt.
Die Resultate sind in der nachstehenden Tabelle VI zusammengestellt.

Tabelle VI

| Gewebeprobe | Wasseraufnahme in % |
|---|---|
| - nicht ausgerüstet | 92,3 |
| - ausgerüstet und bestrahlt | 23,1 |
| - ausgerüstet aber nicht bestrahlt | 79,8 |

Beispiel 24:

Ein Baumwoll-Cretonnegewebe (135 g/m²) wird mit einer Flotte enthaltend
50 g des Polymerisates von Beispiel 14,
950 g Dioxan und
0,01 g eines Sensibilisators
imprägniert, auf eine Flottenaufnahme von 85 % abgequetscht und danach bei 60°C getrocknet. Das behandelte trockene Gewebe wird nun beidseitig, wie in Beispiel 22 beschrieben, bestrahlt und anschliessend zwei Mal mit Dioxan extrahiert, um gegebenenfalls nicht reagiertes Polymerisat zu entfernen.

Von diesem Gewebe wird die Biegefestigkeit gemäss der Vorschrift ASTM D 1388-64 T bestimmt.

Der gleiche Test wird an einer nicht ausgerüsteten und an einer ausgerüsteten aber nicht bestrahlten Gewebeprobe durchgeführt.

Die Resultate sind in der nachstehenden Tabelle VII zusammengestellt.

Tabelle VII

| Gewebeprobe | Biegesteifheit* |
|---|---|
| - nicht ausgerüstet | 457 |
| - ausgerüstet und bestrahlt | 6487 |
| - ausgerüstet aber nicht bestrahlt | 630 |

* Je grösser die Zahl um so steifer die Probe.

Beispiel 25:

Man verfährt wie in Beispiel 23 beschrieben, verwendet jedoch ein 33/67 Baumwolle/Polyestergewebe (290 g/m²) und eine Flotte enthaltend
100 g des Polymerisates von Beispiel 18,
900 g Dioxan und
0,02 g eines Sensibilisators
Das so behandelte Gewebe wird dann gemäss DIN 53886 auf seine Wasserdichtheit hin geprüft.

Der gleiche Test wird an einer nicht ausgerüsteten und an einer ausgerüsteten aber nicht bestrahlten Gewebeprobe durchgeführt. Die Wasserdichtigkeit wird in cm Wassersäule ausgedrückt.

Die Resultate sind in der nachstehenden Tabelle VIII zusammengefasst.

Tabelle VIII

| Gewebeprobe | cm Wassersäule |
|---|---|
| - nicht ausgerüstet | 0 |
| - ausgerüstet und bestrahlt | 13 |
| - ausgerüstet aber nicht bestrahlt | 0 |

## Patentansprüche

1. N-Alkenoyl-enamide der Formel

(1) 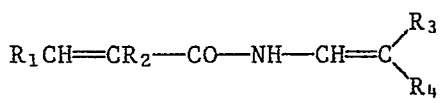

worin
$R_1$ Wasserstoff oder Methyl,
$R_2$ Wasserstoff, $C_1$-$C_4$-Alkyl oder Halogen und
$R_3$ und $R_4$ unabhängig voneinander $C_1$-$C_4$-Alkanoyl, $C_1$-$C_{18}$-Alkoxycarbonyl, Benzoyl, Phenyl, Cyano oder Carbamoyl und $R_4$ auch Wasserstoff bedeuten.

2. N-Alkenoyl-enamide gemäss Anspruch 1, worin $R_2$ Wasserstoff, Methyl oder Chlor und $R_3$ und $R_4$ unabhängig voneinander Acetyl, $C_1$-$C_{18}$-Alkoxycarbonyl, Benzoyl, Phenyl, Cyano oder Carbamoyl und $R_4$ auch Wasserstoff bedeuten.

3. N-Alkenoyl-enamide gemäss Anspruch 2, worin $R_2$ Wasserstoff oder Methyl und $R_3$ und $R_4$ unabhängig voneinander Acetyl oder Ethoxycarbonyl bedeuten.

4. Verfahren zur Herstellung von N-Alkenoyl-enamiden der Formel

(1) 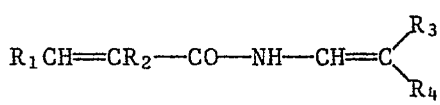

worin
$R_1$ Wasserstoff oder Methyl,
$R_2$ Wasserstoff, $C_1$-$C_4$-Alkyl oder Halogen und
$R_3$ und $R_4$ unabhängig voneinander $C_1$-$C_4$-Alkanoyl, $C_1$-$C_{18}$-Alkoxycarbonyl, Benzoyl, Phenyl, Cyano oder Carbamoyl und $R_4$ auch Wasserstoff bedeuten, dadurch gekennzeichnet, dass man ein Alkenamidderivat der Formel

$$R_1CH = CR_2-CO-NH_2$$

worin $R_1$ und $R_2$ die oben angegebene Bedeutung haben mit einem Enolether der Formel

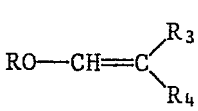

worin $R_3$ und $R_4$ die oben angegebene Bedeutung haben und R für $C_1$-$C_4$-Alkyl steht, in Anwesenheit eines alkanolabspaltenden Katalysators umsetzt.

5. Verfahren gemäss Anspruch 4, worin als Katalysator Phenothiazin verwendet wird.

6. Verfahren gemäss Anspruch 5, dadurch gekennzeichnet, dass die Umsetzung bei einer Temperatur von 130 bis 200, vorzugsweise 140 bis 190°C vorgenommen wird.

7. Verwendung der gemäss dem Verfahren nach Anspruch 4 hergestellten N-Alkenoyl-enamide zur Herstellung von Polymeren, enthaltend, bezogen auf das Gesamtpolymere, 100-1 Mol % an Struktureinheiten der Formel I und 0-99 Mol % an Struktureinheiten der Formel II

(I) 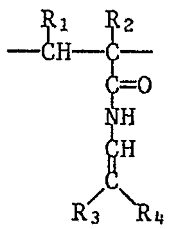 , (II) 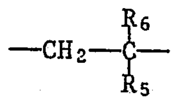

worin

$R_1$, $R_2$, $R_3$ und $R_4$ die Anspruch 4 angegebenen Bedeutungen haben und $R_5$ für Carboxy, $C_1$-$C_{18}$-Alkoxycarbonyl, N-Di-$C_1$-$C_4$-alkylamino-$C_1$-$C_4$-alkylcarbamoyl Cyano, Formyl, N,N-Di-$C_1$-$C_4$-alkylamino-$C_1$-$C_4$-alkoxycarbonyl, $C_1$-$C_4$-Alkanoyloxy, unsubstituiertes oder substituiertes Carbamoyl, 1-Pyrrolidonyl, 1-Imidazolyl, 2- bzw. 4-Pyridinyl oder Phenyl und $R_6$ für Wasserstoff oder Methyl stehen, durch radikalinduzierte Polymerisation in Gegenwart eines organischen inerten Lösungsmittels und eines Polymerisationsinitiators bei erhöhter Temperatur.

8. Verwendung gemäss Anspruch 7, dadurch gekennzeichnet, dass Ethylacetat, Tetrahydrofuran oder Dioxan als Lösungsmittel für die Polymerisation verwendet wird.

9. Verwendung gemäss einem der Ansprüche 7 und 8, dadurch gekennzeichnet, dass tert.-Butylperoxi-2-ethylhexanoat oder Didecanoylperoxid als Polymerisationsinitiator verwendet wird.

10. Verwendung gemäss einem der Ansprüche 7 bis 9, dadurch gekennzeichnet, dass die Polymerisation bei einer Temperatur von 60-100°C vorgenommen wird.

11. Verwendung der Polymere gemäss Anspruch 7 für in Photolacken einsetzbare, strahlenchemisch vernetzbare Massen sowie als UV-Absorber.

12. Verwendung der Polymere gemäss Anspruch 7 als Textilveredelungsmittel.

13. Polymere enthaltend, bezogen auf das Gesamtpolymere, 100-1 Mol % an Struktureinheiten der Formel I und 0-90 Mol % an Struktureinheiten der Formel II

(I) 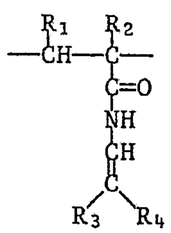 , (II) 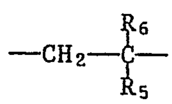

worin

$R_1$ Wasserstoff oder Methyl,

$R_2$ Wasserstoff, $C_1$-$C_4$-Alkyl oder Halogen und

$R_3$ und $R_4$ unabhängig voneinander $C_1$-$C_4$-Alkanoyl, $C_1$-$C_{18}$-Alkoxycarbonyl, Cyano oder Carbamoyl und $R_4$ auch Wasserstoff bedeuten und $R_5$ für Carboxy, $C_1$-$C_{18}$Alkoxycarbonyl, N-Di-$C_1$-$C_4$-alkylamino-$C_1$-$C_4$-alkylcarbamoyl Cyano, Formyl, N,N-Di-$C_1$-$C_4$-alkylamino-$C_1$-$C_4$-alkoxycarbonyl, $C_1$-$C_4$-Alkanoyloxy, unsubstituiertes oder substituiertes Carbamoyl, 1-Pyrrolidonyl, 1-Imidazolyl, 2- bzw. 4-Pyridinyl oder Phenyl und $R_6$ für Wasserstoff oder Methyl stehen.

Europäisches Patentamt

# EUROPÄISCHER RECHERCHENBERICHT

Nummer der Anmeldung

EP 88810501.2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A1 - 0 218 089 (BAYER AG)<br>* Ansprüche *<br>-- | 1,4,7 | C 07 C 103/58<br>C 07 C 102/00<br>C 08 F 20/56 |
| D,A | DE - A1 - 2 718 552 (SUMITOMO)<br>* Ansprüche *<br>-- | 1,4 | |
| A | US - A - 3 657 340 (F.JOHNSON et al.)<br>* Ansprüche *<br>---- | 1,4 | RECHERCHIERTE SACHGEBIETE (Int. Cl.4)<br>C 07 C 103/00<br>C 08 F 20/00<br>C 08 F 120/00<br>C 08 F 220/00 |

Der vorliegende Recherchenbericht wurde für alle Patentansprüche erstellt.

| Recherchenort | Abschlußdatum der Recherche | Prüfer |
|---|---|---|
| WIEN | 04-11-1988 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82